(19) Europäisches Patentamt
European Patent Office
Office européen des brevets

(11) Veröffentlichungsnummer: **0 259 850 B1**

(12) # EUROPÄISCHE PATENTSCHRIFT

(45) Veröffentlichungstag der Patentschrift: **17.07.91**

(51) Int. Cl.⁵: **C07C 239/20**

(21) Anmeldenummer: **87113161.1**

(22) Anmeldetag: **09.09.87**

(54) **Verfahren zur Herstellung von O-substituierten Hydroxylamin-Hydrochloriden.**

(30) Priorität: **12.09.86 DE 3631071**

(43) Veröffentlichungstag der Anmeldung:
**16.03.88 Patentblatt 88/11**

(45) Bekanntmachung des Hinweises auf die Patenterteilung:
**17.07.91 Patentblatt 91/29**

(84) Benannte Vertragsstaaten:
**AT BE CH DE FR GB IT LI NL**

(56) Entgegenhaltungen:

**HOUBEN-WEYL: "Methoden der organischen Chemie", Band X/1, 1971, Georg Thieme Verlag, Stuttgart;**

**CHEMICAL ABSTRACTS, Band 67, Nr. 20, 13. November 1967, Seite 8718, Spalte 1, Zusammenfassung Nr. 92406w, Columbus, Ohio, US; F. MIKULA et al.: "Production of high purity crystalline hydroxylamine hydrochloride" & CHEM. PRUM. 1967, 17(8), 415-417**

**CHEMICAL ABSTRACTS, Band 96, Nr. 16, April 1982, Seite 696, Spalte 1, Zusammenfassung Nr. 142207d, Columbus, Ohio, US; A.D. ZORINA et al.: "Method for the synthesis O-carboxymethylhydroxylamine hydrochloride", & ZH. OBSHCH. KHIM. 1982, 52(1), 223-224**

(73) Patentinhaber: **BASF Aktiengesellschaft**
**Carl-Bosch-Strasse 38**
**W-6700 Ludwigshafen(DE)**

(72) Erfinder: **Will, Wolfgang, Dr.**
**Im Buegen 12**
**W-6719 Kirchheim(DE)**
Erfinder: **Faust, Tillmann, Dr.**
**Parkweg 4**
**W-6714 Weisenheim(DE)**
Erfinder: **Schaefer, Peter, Dr.**
**Im Buegen 16**
**W-6719 Kirchheim(DE)**
Erfinder: **Hartmann, Horst**
**Lindenstrasse 45**
**W-6737 Boehl-Iggelheim(DE)**

EP 0 259 850 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von O-substituierten Hydroxylamin-Hydrochloriden der Formel I

$R\text{-}O\text{-}NH_2 \times HCl$ (I),

in der R eine $C_1$-$C_4$-Alkylgruppe, eine $C_3$- oder $C_4$-Alkenylgruppe, eine $C_3$- oder $C_4$-Halogenalkenylgruppe oder die Benzylgruppe bedeutet.

Zur Herstellung von O-substituierten Hydroxylaminen sind zahlreiche Methoden bekannt, die jedoch in technischer und wirtschaftlicher Hinsicht nicht befriedigen.

Speziell ist aus Houben-Weyl, "Methoden der Organischen Chemie", Band 10/1, 4. Auflage, 1971, 1186-1189 bekannt, bestimmte Aldoxim- und Ketoximether mit Mineralsäuren zu den entsprechenden Salzen des O-substituierten Hydroxylamins zu hydrolysieren, jedoch lassen die dabei erhältichen Ausbeuten zu wünschen übrig.

Der Erfindung lag daher die Aufgabe zugrunde, die für die Synthese von Arzneimitteln und Pflanzenschutzmitteln wichtigen O-substituierten Hydroxylamine I technisch und wirtschaftlich besser zugänglich zu machen.

Demgemäß wurde ein Verfahren zur Herstellung von O-substituierten Hydroxylamin-Hydrochloriden der allgemeinen Formel I

$R\text{-}O\text{-}NH_2 \times HCl$ (I)

gefunden, in der R eine $C_1$-$C_4$-Alkylgruppe, eine $C_3$- oder $C_4$-Alkenylgruppe, eine $C_3$- oder $C_4$-Halogenalkenylgruppe oder die Benzylgruppe bedeutet, durch Spaltung des entsprechenden Acetonoximethers der allgemeinen Formel II

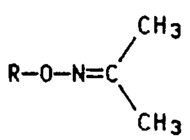

(II),

wobei man erfindungsgemäß die Spaltung mit Chlorwasserstoff und Wasser in einer Reaktionskolonne mit mindestens 20 theoretischen Böden unter laufender Entfernung des hierbei abgespaltenen Acetons kontinuierlich vornimmt.

Der Rest R bedeutet beispielsweise:

- eine $C_1$-$C_4$-Alkylgruppe, d.h. Methyl, Ethyl, n-Propyl, iso-Propyl, n-Butyl, iso-Butyl, sec.-Butyl und tert.-Butyl,
- eine $C_3$- oder $C_4$-Alkenylgruppe, wie Prop-2-enyl, But-2-enyl und But-3-enyl,
- eine $C_3$- oder $C_4$-Halogenalkenylgruppe, wie 3-Chlorprop-2-enyl, 2-Chlorprop-2-enyl, 2-Chlorbut-2-enyl, 3-Chlorbut-2-enyl, 2,3-Dichlorprop-2-enyl, 2,3-Dichlorbut-2-enyl, 3-Bromprop-2-enyl, 3-Fluorprop-2-enyl, 2-Bromprop-2-enyl, 2-Fluorprop-2-enyl, 2,3-Dibromprop-2-enyl und 2,3-Dibrombut-2-enyl oder
- die Benzylgruppe.

Die Ausgangsverbindungen II sind bekannt oder nach bekannten Methoden erhältlich, z.B. durch Veretherung des Acetonoxims (DE-OS 29 27 117, EP-A-121 701, EP-A-158 159).

Zu der Umsetzung der Acetonoximether II zu den O-substituierten Hydroxylamin-Hydrochloriden I ist das folgende zu sagen:

Als Reaktionskolonnen eignen sich besonders Bodenkolonnen aller Bauarten, vor allem Glockenbodenkolonnen, da sich in ihnen die Verweilzeiten auf den Böden gut einstellen lassen. Füllkörperkolonnen kommen auch in Betracht. Die Zahl der theoretischen Böden liegt i.a. zwischen 20 und 60, bevorzugt werden 30 bis 50 theoretische Böden, jedoch ist die Bodenzahl nach oben nur durch wirtschaftliche Überlegungen begrenzt.

Den für die Hydrolyse erforderlichen Chlorwasserstoff setzt man vorzugsweise in stöchiometrischer Menge ein, jedoch ist auch die Verwendung eines Überschusses - etwa bis zu 1 mol je mol Acetonoximether II - möglich.

Verwendet man wäßrige Salzsäure, beträgt deren Konzentration vorzugsweise 10 bis 20, insbesondere

15 bis 20 Gew.%.

Man kann nach einer besonderen Ausführungsform in Gegenwart von 1,4-Dioxan arbeiten, aus dem sich das Hydroxylamin I gut kristallin gewinnen läßt. Allerdings muß man in diesem Fall so wenig Wasser wie möglich (wenig über 1 mol) und d.h. auch Chlorwasserstoff gasförmig verwenden.

Im übrigen leitet man den Reaktionspartner II, eine Lösung von II, Wasser und Chlorwasserstoff bzw. wäßrige Salzsäure, zweckmäßigerweise in den Mittelteil der Kolonne ein und richtet es durch Wahl der Zulaufgeschwindigkeit und der Heizleistung so ein, daß die mittlere Verweilzeit in der Kolonne 3 bis 4 Stunden beträgt. Für die Einleitungsstelle gilt allgemein die Regel, daß Verbindungen II mit höherem Siedepunkt auf einem höheren Boden zugegeben werden als solche mit niedrigem Siedepunkt. Die Reaktionstemperatur liegt zweckmäßigerweise zwischen 70 und 140 °C, so daß man bei Atmosphärendruck arbeiten kann; erforderlichenfalls bei geringem Unterdruck bis ca. 500 mbar oder bei geringem Überdruck bis etwa 3 bar. Das kontinuierlich mit einem Rücklaufverhältnis von z.B. 1:5 bis 1:50, vorzugsweise 1:5 bis 1:20, über Kopf abgezogene Aceton kann zur Herstellung von Acetonoximether II wiederverwendet werden.

Im Sumpf der Kolonne fällt eine wäßrige Lösung von O-substituiertem Hydroxylamin-Hydrochlorid I bzw. eine Suspension von I in 1,4-Dioxan an.

Man trägt diese Lösung bzw. Suspension kontinuierlich aus und arbeitet sie, falls erforderlich, wie üblich durch Kristallisation oder Abziehen der Flüssigkeit auf die reinen Verfahrensprodukte I auf.

Hierbei fallen die Verbindungen I in hoher Ausbeute und in einer Reinheit an, die sie unmittelbar für Synthesen einsetzbar machen.

Die O-substituierten Hydroxylamin-Hydrochloride sind wertvolle Zwischenprodukte zur Herstellung von Arzneimitteln und Pflanzenschutzmitteln.

Herstellung der O-substituierten Hydroxylamin-Hydrochloride I

Beispiel 1

Am 30. Boden einer Glockenbodenkolonne mit 60 praktischen Böden und 50 mm Innendurchmesser, die mit einem Dünnfilmverdampfer und einem automatischen Rücklaufteiler ausgestattet ist, wurden pro h 131,2 g Acetonoximethylether in 237,5 g 1,4-Dioxan, 31,3 g Wasser und 47,3 g Chlorwasserstoff zudosiert. Aceton wurde bei einem Rücklaufverhältnis von 1:15 über Kopf abgenommen und das Dioxan-Produkt-Gemisch als Sumpf. Letzteres wurde abgesaugt und der Feststoff im Vakuum getrocknet. Man erhielt 123,0 g/h (97 % Ausbeute) Ethoxyaminhydrochlorid (Verbindung 1), Fp. 133 °C.

Beispiel 2

In der vorstehend beschriebenen Anlage wurden am 50. Boden 139,9 g/h Acetonoxim-trans-crotylether und 315 g/h 1,4-Dioxan, am 20. Boden 40,1 g/h Chlorwasserstoff und am 30. Boden 25,8 g/h Wasser zudosiert. Aceton wurde bei einem Rücklaufverhältnis von 1:16 über Kopf abgenommen. Nach dem Auskühlen wurde das Produkt abgesaugt und im Vakuum getrocknet.

Man erhielt 126,4 g/h (93 % Ausbeute) trans-Crotyl-oxyamin-hydrochlorid (Verbindung 2), Fp. 169 °C.

Analog zu Beispiel 1 und 2 wurden die in Tabelle 1 aufgeführten O-substituierten Hydroxylamin-Hydrochloride aus den entsprechenden Acetonoximen II erhalten:

Tabelle 1

$H_2N\text{-}O\text{-}R \times HCl$ (I)

| Verbindung | R | Fp. [°C] | Ausbeute % |
|---|---|---|---|
| 3 | $CH_3$ | 150 | 96 |
| 4 | $(CH_2)_2CH_3$ | 154 | 95 |
| 5 | $(CH_2)_3CH_3$ | 153 | 93 |
| 6 | $CH_2CH(CH_3)_2$ | 129 | 92 |
| 7 | $(CH_2)_4CH_3$ | 149 | 91 |
| 8 | $CH_2CH{=}CH_2$ | 168 | 94 |
| 9 | trans-$CH_2\text{-}CH{=}CHCl$ | 180 | 92 |
| 10 | $CH_2C(CH_3){=}CH_2$ | 165 | 91 |
| 11 | $CH_2C_6H_5$ | 225 | 91 |

Beispiel 3

In die in Beispiel 1 beschriebene Glockenbodenkolonne wurden am 10. Boden 113,3 g/h Acetonoximmethylether und am 40. Boden 262,8 g/h 18 %ige wäßrige Salzsäure zudosiert. Aceton wurde bei einem Rücklaufverhältnis von 1:8 über Kopf abgenommen, die wäßrige Lösung des Produkts als Sumpf. Man erhielt 300,9 g/h einer 35 %igen Lösung von Methoxyamin-hydrochlorid (Verbindung 3) (≅ 105,3 g/h Trockensubstanz, 97 % Ausbeute).

Analog zu Beispiel 3 wurden die in Tabelle 2 aufgeführten O-substituierten Hydroxylamine I in Form ihrer Hydrochloride in wäßriger Lösung aus den entsprechenden Acetonoximethern II erhalten:

Tabelle 2

$[H_2N\text{-}O\text{-}R \times HCl]_{aq}$ (I)

| Verbindung | R | Ausbeute % |
|---|---|---|
| 1 | $CH_2CH_3$ | 97 |
| 4 | $CH_2CH_2CH_3$ | 95 |
| 8 | $CH_2CH{=}CH_2$ | 93 |

**Patentansprüche**

**1.** Verfahren zur Herstellung von O-substituierten Hydroxylamin-Hydrochloriden der allgemeinen Formel I

$R\text{-}O\text{-}NH_2 \times HCl$ (I),

in der R eine $C_1$-$C_4$-Alkylgruppe, eine $C_3$- oder $C_4$-Alkenylgruppe, eine $C_3$- oder $C_4$-Halogenalkenylgruppe oder die Benzylgruppe bedeutet, durch Spaltung eines entsprechenden Acetonoximethers der allgemeinen Formel II

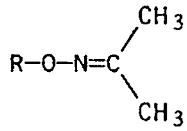 (II),

dadurch gekennzeichnet, daß man die Spaltung mit Chlorwasserstoff und Wasser in einer Reaktionskolonne mit mindestens 20 theoretischen Böden unter laufender Entfernung des hierbei abgespaltenen Acetons kontinuierlich vornimmt.

2. Verfahren nach Anspruch 1, dadurch gekennzeichnet, daß man die Spaltung mit einer stöchiometrischen Menge von Chlorwasserstoff und Acetonoximether II vornimmt.

3. Verfahren nach den Ansprüchen 1 und 2, dadurch gekennzeichnet, daß man den Chlorwasserstoff in Form von 10 bis 20 gew.%iger Salzsäure einsetzt.

4. Verfahren nach Ansprüchen 1 und 2, dadurch gekennzeichnet, daß man die Hydrolyse in 1,4-Dioxan als Lösungsmittel, mit 1 bis 1,6 mol Wasser pro Mol Acetonoximether II und unter Einleiten von Chlorwasserstoffgas vornimmt.

**Claims**

1. A process for the preparation of an O-substituted hydroxylamine hydrochloride of the formula I

$R\text{-}O\text{-}NH_2 \cdot HCl$ (I)

where R is $C_1$-$C_4$-alkyl, $C_3$- or $C_4$-alkenyl, $C_3$- or $C_4$-haloalkenyl or benzyl, by cleaving a corresponding acetoxime ether of the formula II

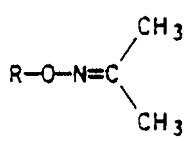 (II),

wherein the cleavage is carried out continuously using hydrogen chloride and water in a reaction column containing not less than 20 theoretical plates with constant removal of the acetone eliminated.

2. A process as claimed in claim 1, wherein the cleavage is carried out using a stoichiometric amount of hydrogen chloride and acetoxime ether II.

3. A process as claimed in claims 1 and 2, wherein the hydrogen chloride is used in the form of 10-20% strength by weight hydrochloric acid.

4. A process as claimed in claims 1 and 2, wherein the hydrolysis is carried out in 1,4-dioxane as the solvent, with from 1 to 1.6 moles of water per mole of acetoxime ether II and while passing in hydrogen chloride gas.

**Revendications**

1. Procédé de préparation de chlorhydrates d'hydroxylamines O-substituées de formule générale I

$R\text{-}O\text{-}NH_2 \times HCl$ (I)

dans laquelle R représente un groupement alkyle en $C_1$-$C_4$,un groupement alcényle en $C_3$ ou $C_4$, un

groupement halogénoalcényle en $C_3$ ou $C_4$ ou le groupement benzyle, par coupure d'un acétonoxime-éther correspondant de formule générale II

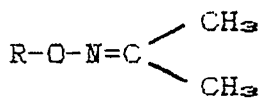

(II)

caractérisé en ce qu'on procède en continu à la coupure avec de l'acide chlorhydrique et de l'eau dans une colonne de réaction comportant au moins 20 plateaux théoriques, avec élimination continue de l'acétone ainsi séparée.

**2.** Procédé selon la revendication 1, caractérisé en ce qu' on procède à la coupure avec une quantité stoechiométrique d'acide chlorhydrique et d'acétonoxime-éther II.

**3.** Procédé selon la revendication 1 ou 2, caractérisé en ce qu'on utilise l'acide chlorhydrique sous forme d'acide chlorhydrique aqueux à 10 à 20% en poids.

**4.** Procédé selon la revendication 1 ou 2, caractérisé en ce qu'on procède à l'hydrolyse dans du 1,4-dioxanne en tant que solvant, avec 1 à 1,6 mole d' eau par mole d' acétonoxime-éther II et en faisant passer du gaz chlorhydrique.